# EUROPÄISCHE PATENTANMELDUNG

(11) **EP 0 739 617 A2**
(43) Veröffentlichungstag der Anmeldung: **30.10.1996**
(21) Anmeldenummer: 96103062.4
(22) Anmeldetag: 29.02.1996
(51) Int. Cl.: A61F 13/10

(54) **Selbstklebende Fertigbandage für Ellenbogen**

(30) Priorität: 31.03.1995 DE 19512013
(71) Anmelder: Beiersdorf Aktiengesellschaft, D-20245 Hamburg (DE)
(72) Erfinder: Staudinger, Peter, 25436 Tornesch (DE)
(74) Vertreter: Dinné, Erlend

(57) **Zusammenfassung**

Auf einer Seite selbstklebend beschichtete Fertigbandage zur Stützung und partiellen Fixierung des Ellenbogengelenks, die aus einem länglichen Streifen besteht, der einseitig in Längsrichtung bis etwa zur Mitte des Streifens einen Einschnitt aufweist. Vorzugsweise besteht sie aus einem längselastischen Gewebe oder Gewirke.

## Beschreibung

Die Erfindung betrifft eine einseitig selbstklebend beschichtete Fertigbandage zur Stützung, Fixierung, Stabilisierung und Entlastung des Ellbogengelenks.

Die funktionelle Verbandtechnik, das sog. Taping, ist eine gängige Behandlungsmethode zur Prophylaxe und Therapie von Verletzungen, Erkrankungen und Veränderungen am Bewegungsapparat. Taping hat zum Ziel, die einzelnen Weichteile und Kapsel-, Band-Strukturen gezielt nachzubilden bzw. deren Funktionen selektiv zu stützen.

Der eigentliche Tapeverband wird aus mehreren Bändern, sog. Zügeln, streifenweise unter Verwendung von vorzugsweise unelastischem Material, teilweise kombiniert mit elastischem Material, angelegt und erfüllt dann die Funktionen Stützung und Entlastung.

Derartige Verbände erfordern jedoch fachmännisches Können und sehr viel Erfahrung und können deshalb in aller Regel nicht vom Taping Unerfahrenen angelegt werden.

Aufgabe der Erfindung war es deshalb, eine Fertigbandage zur Verfügung zu stellen, die aufgrund ihrer Ausgestaltung, ihres Materials sowie der selbstklebenden Eigenschaften, die Anforderungen eines Volltapeverbandes weitgehend erfüllt und darüber hinaus vom Verwender in einfacher und schneller Weise angelegt werden kann.

Gelöst wird diese Aufgabe durch eine Fertigbandage gemäß Anspruch 1.

Der längliche Streifen ist insgesamt etwa 1 m lang, vorzugsweise etwa 90 cm, und etwa 8 cm breit, wobei der ungeteilte Abschnitt etwa 50 cm und der geteilte Abschnitt etwa 40 cm lang ist. Der Trennschnitt wird in der Regel mittig in den Streifen eingeführt, so daß die dadurch entstehenden beiden schmalen Streifen etwa 4 cm breit sind.

Die Bandage besteht vorzugsweise aus einem längselastischen Gewebe oder Gewirke, das gegebenenfalls auch eine geringe Querelastizität aufweisen kann, insbesondere auf Baumwollbasis. Die Längselastizität entspricht vorzugsweise derjenigen von sog. Kurzzug-Binden, d.h. Binden mit einer Dehnungsfähigkeit von ca. 60% - 90%.

Als sehr günstig hat es sich erwiesen, wenn sich am inneren Ende des Einschnitts eine Ausnehmung in Form eines Dreiecks befindet. Dadurch wird der Sitz der Bandage verbessert und das Anlegen erleichtert. Das Dreieck ist in der Weise angebracht, daß seine Spitze das Ende des Einschnitts darstellt und seine Basis quer zur Längsrichtung der Bandage verläuft. Das Dreieck ist vorzugsweise gleichschenklig und in etwa auch gleichseitig. Seine Basis beträgt etwa ein Drittel der Breite der Bandage.

Ebenfalls vorteilhaft ist es, wenn die Enden der Bandage abgerundet sind. Dies erleichtert das Wiederabziehen der Bandage nach Gebrauch. Gegebenenfalls kann auch nur ein Ende abgerundet sein und zwar insbesondere das des ungeteilten breiten Abschnitts.

Auf ihrer der Haut zugewandten Seite ist die Bandage mit einer der bekannten gut haftenden Selbstklebemasse beschichtet auf Basis von Kautschuk oder synthetischen Polymeren. Vorzugsweise sollten diese luft- und wasserdampfdurchlässig sowie gut hautverträglich sein.

Bis zum Gebrauch der Bandage ist diese Klebeschicht mit einem klebstoffabweisend ausgerüsteten Blattmaterial, wie beispielsweise silikonisiertem Papier oder Kunststoff-Folie, abgedeckt.

Es hat sich dabei als günstig erwiesen, diese Abdeckung mehrteilig, vorzugsweise dreiteilig auszubilden. Dabei deckt ein Teil, ebenfalls streifenförmig, den ungeteilten Abschnitt des Streifens der Bandage und je zwei weitere streifenförmige Teile die schmaleren Streifen der geteilten Seite ab. Die Abdeckungsteile können als Anbringungshilfe farbig markiert oder nummeriert sein.

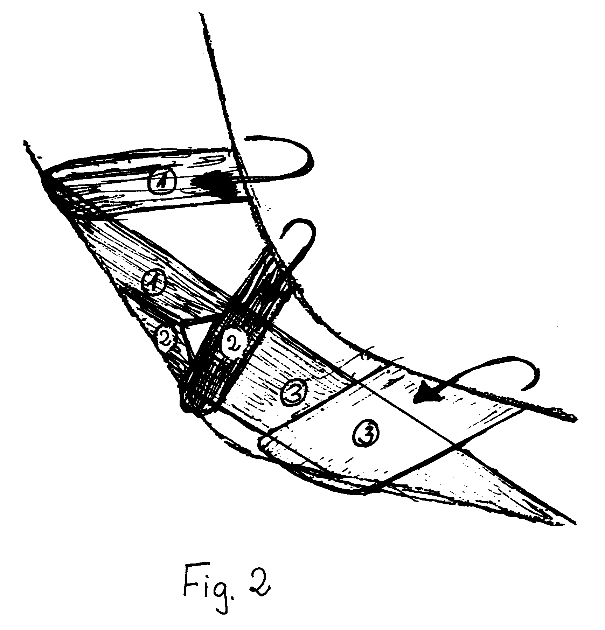

Beim Anlegen der Bandage wird, wie in Figur 2 gezeigt, diese so plaziert, daß die dreieckige Ausnehmung lateral der Ellenbeuge liegt, wobei die Spitze des Dreiecks nach oben zeigt. Zuerst wird das Abdeckpapier vom ungeteilten Abschnitt (3) des Streifens entfernt und dieser von proximal nach distal verlaufend über den Epicondylus zum volaren Unterarm hingeführt und angeklebt. Dann erfolgt mit dem Rest dieses Zügels eine ellipsenförmige Tour um den Unterarm und der Zügel endet an der Außenseite des Ellbogens auf sich selbst und fixiert sich somit selbst. Die ellipsenförmige Tour wird ohne Zug angelegt um Stauungen oder Abschnürungen zu vermeiden.

Die schmalen Zügel des geschlitzten Teiles der Bandage werden nach Entfernen des Abdeckpapiers, zunächst mit dem proximalen Zügel (1) beginnend, um den Oberarm ohne Zug angelegt, wobei der Zügel (1) sich mit seinem Ende günstigstenfalls am Oberarm selbst fixiert.

Der äußere Zügel (2) wird abschließend unterhalb des Zügels (1) um den distalen Oberarm gelegt und endet auf der Außenseite des Ellenbogens günstigenfalls am Epicondylus.

Die Funktionen der einzelnen Zügel lassen sich, wenn sie wie dargestellt angelegt sind, wie folgt beschreiben:

Der schmale Zügel (1) wirkt als Fixationszügel und Extensions einschränkende Verstärkung. Der schmale Zügel (2) dient als Entlastungszügel für den Epicondylus und der breite Zügel (3) als Extensions einschränkender Zügel am Unterarm.

Insgesamt gesehen schützt, fixiert, stabilisiert und entlastet damit die angelegte Bandage das Ellenbogengelenk in funktionell anatomisch angepaßter Weise.

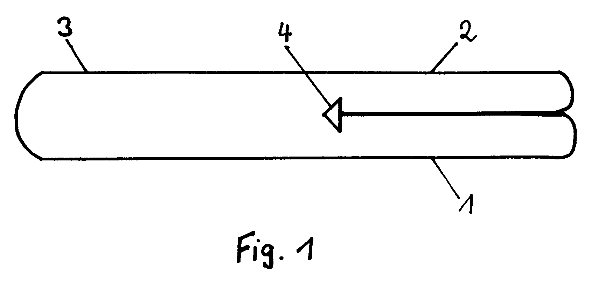

Die erfindungsgemäße Bandage ist in Figur 1 beispielsweise dargestellt. Dabei bedeuten (1) und (2) die beiden schmalen Zügel des geteilten Abschnitts und (3) den breiten Zügel des ungeteilten Abschnitts. Mit (4) ist die dreieckige Ausnehmung bezeichnet.

Die Figur 2 zeigt die Bandage in angelegtem Zustand, wobei die Pfeile die Wickelrichtung angeben.

## Patentansprüche

1. Auf einer Seite selbstklebend beschichtete Fertigbandage zur Stützung und partiellen Fixierung des Ellgenbogengelenks, dadurch gekennzeichnet, daß sie aus einem länglichen Streifen besteht, der einseitig in Längsrichtung bis etwa zur Mitte des Streifens einen Einschnitt aufweist.

2. Selbstklebende Bandage gemäß Anspruch 1, dadurch gekennzeichnet, daß der ungeteilte Abschnitt (3) des Streifens etwa 50 cm lang und 8 cm breit ist und die durch den Einschnitt in dem andereren Teil des Streifens entstandenen beiden schmalen Streifen (1) und (2) etwa 40 cm lang und je 4 cm breit sind.

3. Selbstklebende Bandage gemäß Anspruch 1 oder 2, dadurch gekennzeichnet, daß sie aus einem längselastischen Gewebe oder Gewirke besteht.

4. Selbstklebende Bandage gemäß einem der Ansprüche 1 bis 3, dadurch gekennzeichnet, daß sich am inneren Ende des Einschnitts eine Ausnehmung in Form eines Dreiecks (4) befindet, an dessen Spitze der Einschnitt endet und dessen Basis quer zur Längsrichtung der Bandage verläuft.

5. Selbstklebende Bandage gemäß Anspruch 4, dadurch gekennzeichnet, daß die Basis des Dreiecks (4) etwa 1/3 der Breite der Bandage beträgt.

6. Selbstklebende Bandage gemäß einem der Ansrpüche 1 bis 5, dadurch gekennzeichnet, daß die Enden der Streifen (1), (2) und (3) abgerundet sind.

7. Selbstklebende Bandage gemäß einem der Ansprüche 1 bis 5, dadurch gekennzeichnet, daß sie auf ihrer selbstklebenden Seite mit klebstoffabweisendem Material abgedeckt ist.

8. Selbstklebende Bandage gemäß Anspruch 7, dadurch gekennzeichnet, daß das klebstoffabweisende Material dreiteilig ausgebildet ist, wobei ein Teil den ungeteilten Abschnitt (3) abdeckt und je ein weiteres Teil die schmalen Streifen (1) und (2).
